# EUROPEAN PATENT APPLICATION

(11) **EP 2 042 607 A1**
(43) Date of publication of application: **01.04.2009**
(21) Application number: 07790983.6
(22) Date of filing: 19.07.2007
(51) Int. Cl.: C12P 7/64, A23D 7/00, A23G 1/00, A23G 1/30

(54) **PROCESS FOR PRODUCTION OF HARD BUTTER SUITABLE FOR CHOCOLATE PRODUCT**

(30) Priority: 19.07.2006 JP 2006196624
(71) Applicant: The Nisshin Oillio, Ltd., Tokyo 104-8285 (JP)
(72) Inventor: UEHARA, Hidetaka, Yokosuka-shi Kanagawa 239-0832 (JP); ARIMOTO, Shin, Yokosuka-shi Kanagawa 239-0832 (JP); SUGANUMA, Tomomi, Yokosuka-shi Kanagawa 239-0832 (JP); NEGISHI, Satoshi, Yokosuka-shi Kanagawa 239-0832 (JP); SUZUKI, Junko, Yokosuka-shi Kanagawa 239-0832 (JP); YAMAUCHI, Yoshie, Yokosuka-shi Kanagawa 239-0832 (JP); TAKAHASHI, Isamu, Yokosuka-shi Kanagawa 239-0832 (JP); MANABE, Tamami, Yokosuka-shi Kanagawa 239-0832 (JP)
(74) Representative: Bawden, Peter Charles
(86) International application number: PCT/JP2007/064229
(87) International publication number: WO 2008/010543

(57) **Abstract**

There is provided a process for preparing a fat and oil having a 1,3-disaturated long-chain fatty acid 2-monounsaturated long-chain fatty acid triglyceride (SUS)/1,2-disaturated long-chain fatty acid 3-monounsaturated long-chain fatty acid triglyceride (SSU) mass ratio of 9/1 or more, which comprises conducting transesterification of a fat and oil containing SUS of 50% by mass or more and SSU with an action of a 1,3-selective lipase selected from the group consisting of lipases derived from *Thermomyces* sp., *Rhizopus oryzae* and *Rhizopus delemar*. This process is an industrially suitable process for preparing a hard butter which has excellent properties as a cacao butter equivalent (CBE) or cacao butter improver (CBI).

## Description

### Technical Field of the Invention

The present invention relates a process for preparing a hard butter, particularly a hard butter which is excellent as a cacao butter equivalent (CBE) or cacao butter improver (CBI) and suitable for chocolate products.

### Background of the Invention

Hard butter including cacao butter is used extensively for foods in the fields of confectionary production and bread making, which primarily includes chocolate, and pharmaceuticals, cosmetics and the like. Such hard butter comprises, as a primary component, symmetric triglycerides (SUS) having one unsaturated bond in the molecule such as 1,3-dipalmitoyl-2-oleoyl glycerin (POP), 1-palmitoyl-2-oleoyl-3-stearoyl glycerin (POSt) and 1,3-distearoyl-2-oleoyl glycerin (StOSt).

In general, symmetric triglycerides are available as natural fats and oils containing such components, for example, palm oil, shea butter, sal fat, mowra butter and the like or fractioned oils thereof. Among these, palm oil is used as a fat and oil rich in POP, mowra butter is used as a fat and oil rich in POS, and shea butter, sal fat and the like are used as a fat and oil rich in SOS. For a hard butter such as cacao butter equivalent, these fats and oils are usually used directly or by blending in an appropriate manner. However, since shea butter, sal fat, mowra butter and the like are obtained from wild plants, the yield and the price thereof may significantly vary depending on factors such as weather. In the worst case, there is a problem that required quantity of such fat and oil is not ensured.

Palm midfraction (PMF) used as a fat and oil rich in POP is a raw material which can be stably supplied, although it comprises asymmetric triglyceride PPO along with symmetric triglyceride POP (POP/PPO = 84/16). In this connection, it has been known that a high PPO content makes physical properties of chocolates worse, thus processes for removing PPO have ever been proposed. As a process for removing PPO, there is known solvent fractionation with a solvent such as acetone. However, this process is difficult to control the ratio of POP/PPO and also to improve POP/PPO to be 95/5 or more under conditions substantially used.

Patent literature 1 proposes a process for obtaining a cacao butter equivalent, characterized by conducting transesterification of PMF added palmitic acid thereto with a lipase, removing the enzyme and reaction solvent therefrom, deacidifying with a film molecular still, purifying by column chromatography, fractionating with a solvent to obtain a middle melting point fraction, and subjecting the fraction to deodorization treatment by steam stripping under reduced pressure. However, there is a problem that this process has complicate production steps.

Patent literature 2 discloses a process for preparing an improved confectionery fat, characterized by rearranging single edible fat and oil substantially consisting of saturated and unsaturated C16 and C18 fatty acid triglycerides or fraction thereof by contact with a 1,3-selective lipase, and fractionating the resultant to increase symmetric disaturated triglycerides. This process is excellent. However, there are some inefficient problems, for example, its production steps are complicate, its reaction time is very long, and a fraction of the first 24 hours must be removed to minimize hydrolysis in the final product, because in this process the raw material fat and oil is dissolved in a solvent such as petroleum ether and passed through alternate layers of cerite and cerite-lipase catalyst moisturized with water in advance, which alternate layers is disclosed in Patent literature 2.

Patent literature 3 discloses a process for modifying a fat and oil,
characterized by applying a lipase having a specificity for the 1,3-positions of triglycerides to a fat and oil containing 50% by mass or more of symmetric triglycerides in which saturated long-chain fatty acid residues bond to the 1,3-positions and an unsaturated long-chain fatty acid residue binds to the 2-position to reduce an amount of asymmetric triglycerides in which saturated long-chain fatty acid residues bond to the 1,2-positions and unsaturated long-chain fatty acid residue binds to the 3-position. This process is excellent. However, there are problems that the used amount of the immobilized lipase and the reaction time is long due to use of an immobilized lipase derived from *Mucor miehei* which is a lipase having specificity for the 1,3-positions as disclosed in this literature.

Therefore, it is desired to develop more industrially suitable processes for preparing a hard butter as a cacao butter equivalent, which processes can reduce the used amount of the immobilized lipase and have short reaction time.
Patent Literature 1: Japanese Unexamined Patent Publication No. Hei 7-155106
Patent Literature 2: Japanese Examined Patent Publication No. Hei 1-39757
Patent Literature 3: Japanese Unexamined Patent Publication No. Hei 11-169191

### Disclosure of the Invention

An object of the present invention is to provide an industrially suitable process for preparing a hard butter which has excellent properties as a cacao butter equivalent (CBE) or cacao butter improver (CBI).

Palm midfraction comprises 1,3-dipalmitoyl-2-oleoyl glycerin as a primary component and a significant amount of its isomer 1,2-dipalmitoyl-3-oleoyl glycerin (PPO) also referred to as 1-oleoyl-2,3-dipalmitoyl glycerin (OPP). Conducting transesterification in the palm midfraction with an action of a lipase selected from the group consisting of 1,3-selective lipases derived from *Thermomyces* sp., *Rhizopus oryzae* and *Rhizopus delemar*, particularly the lipase in a powdered form, the reaction can be completed in a short time in spite of a little used amount of the lipase, and then substantially removing the resultant monopalmitoyl dioleoyl glycerin (PO₂) and/or tripalmitoyl glycerin (PPP) can yield a fat and oil having an improved POP content, which fat and oil can become a hard butter having excellent properties as a cacao butter equivalent or cacao butter improver by itself. The present invention has been complicated on the basis of these findings.

Namely, the present invention provides a process for preparing a fat and oil having a 1,3-disaturated long-chain fatty acid 2-monounsaturated long-chain fatty acid triglyceride (SUS)/1,2-disaturated long-chain fatty acid 3-monounsaturated long-chain fatty acid triglyceride (SSU) mass ratio of 9/1 or more, which comprises conducting transesterification of a fat and oil containing SUS of 50% by mass or more and SSU with an action of a 1,3-selective lipase selected from the group consisting of lipases derived from *Thermomyces* sp., *Rhizopus ozyzae* and *Rhizopus delemar*.

### Brief Description of Drawing

Fig. 1 shows solid fat contents in the hard butters obtained in Example 4.

### Best Mode for carrying out the Invention

Example of the fat and oil as a raw material used for preparing the hard butter in the present invention includes that containing 1,3-dipalmitoyl-2-oleoyl glycerin (POP) and 1,2-dipalmitoyl-3-oleoyl glycerin (PPO) wherein POP content is 50% by mass or more, preferably 55% by mass or more. Among them, a fat and oil derived from palm oil is preferred, particularly the fat and oil having a POP content of 60% by mass or more is preferred, and more specifically palm midfraction is preferred. Such fat and oil can be obtained by means such as fractionating palm oil.

Here, symbols representing fatty acids are explained below.
S: saturated long-chain fatty acid
U: monounsaturated fatty acid
P: palmitic acid
St: stearic acid
O: oleic acid
L: linoleic acid

Additionally, a brevity code wherein the symbols described above are combined is used as a symbol representing triacylglycerol species. The brevity code formed by writing three symbols representing fatty acids in juxtaposition means triacylglycerol species wherein each of the fatty acids respectively bind to from the sn-1-position toward the sn-3 position of the glycerin backbone. For example, POP means a triacylglycerol wherein palmitic acids bind to the sn-1,3-positions and oleic acid binds to the sn-2-position. In addition, the brevity codes also comprise triacylglycerol species having optical isomer(s) except as otherwise specially described herein. For example, POSt means a sum of POSt and StOP, PPO means a sum of PPO and OPP. When the symbol representing fatty acid has a number indicated by a subscript, the said code means triacylglycerol species in which fatty acids are not particularly specified their binding positions. For example, P₂O means triacylglycerol species to which two palmitic acid and one oleic acid bind regardless of their binding positions, i.e., a sum of PPO, POP and OPP.

It is preferred to use such raw material fat and oil substantially free from high melting point fraction (PPP, P₂St) and low melting point fraction (PO₂, P₂L), because it can omit the step of removing the high melting point fractions and/or low melting point fractions after the reaction. The phrase "substantially free from" means not more than 1% by mass for the high melting point fraction and not more than 7% by mass, preferably not more than 5% by mass for the low melting point fraction. For example, conducting transesterification of a raw material fat and oil substantially free from the low melting point fraction with a lipase derived from *Rhizopus ozyzae* or *Rhizopus delemar* can obtain a hard butter having a significantly improved mass ratio of SUS/SSU simply by dry fractionation to remove the high melting point fraction.

The present invention is characterized by using a lipase selected from the consisting of lipases derived from *Thermomyces* sp., *Rhizopus oryzae* and *Rhizopus delemar* as a 1,3-selective lipase.

As the lipase derived from *Thermomyces* sp., that derived from *Thermomyces lanugenousu* is preferred. It is preferred that these lipases are immobilized on a carrier such as silica, cerite, diatomaceous earth, pearlite and polyvinyl alcohol.

Such immobilized lipases are available, for example, from Novozymes A/S under the trade name of Lipozyme TL-IM.

As a powder of the lipase derived from *Thermomyces* sp., Lipozyme TL(100L) manufactured by Novozymes Japan Ltd is conducted membrane treatment and then powdered by spray drying and the resultant can be used.

However, as a powdered lipase, it is preferred to use a ground product of the lipase derived from *Thermomyces* sp. which is immobilized on a carrier (preferably silica).

It is preferred that the ground product is one ground with a grinder mill so that the ground product has an average particle size of not less than 1 µm and less than 300 µm, preferably 1 to 200 µm, more preferably 1 to 100 µm and especially preferably 20 to 100 µm. In this connection, examples of the grinder mill include mortar, shear friction mill, cutter mill, stone mill (mycolloider, masscolloider), coffee mill, power mill, pin mill, impact grinder (hammer mill, ball mill), roll crusher, air attrition mill, homogenizer, ultrasonic grinder and the like.

In the present invention, furthermore, as the powdered lipase, it is preferred to use a lipase powder composition containing the ground product of the lipase derived from *Thermomyces* sp., immobilized on the silica carrier, which has an average particle size of 1 to 200 µm, preferably 1 to 100 µm and more preferably 20 to 100 µm, and a filter aid. Examples of the filter aid include inorganic filter aids such as cerite and organic filter aids such as fibers e.g., cellulose and their ground products. Among these filter aids, organic filter aids, in particular, organic polymer filter aid is preferred. Among them, cellulose is preferred. Example of preferred cellulose includes that available from NIPPON PAPER CHEMICALS Co., Ltd under the trade name of KC Frock. The filter aid preferably is also in a powder form and has an average particle size of 10 to 90 µm.

A mass ratio of the lipase ground product to the filter aid is preferably 1/10 to 10/1, and particularly the mass ratio is preferred to be 1/7 to 2/1.

The particle size of the powdered lipase can be determined, for example, with Particle Size Distribution Analyzer (LA-500) of HORIBA, Ltd.

In the present invention, *Rhizopus delemar* and *Rhizopus oryzae* belonging to *Rhizopus* genus can be also used.

Examples of a lipase derived from such *Rhizopus* genus include Picantase R8000 (manufactured by Robin), Lipase F-AP15 (manufactured by Amano Enzyme Inc.) and the like, although the most suitable lipase includes Lipase DF "Amano" 15-K (manufactured by Amano Enzyme Inc. and also referred to as Lipase D) derived from *Rhizopus oryzae.* These are powdery lipases. In addition, Lipase DF "Amano" 15-K had been described as a lipase derived from *Rhizopus delemar* in the past.

As the lipase used in the present invention, that obtained by drying a lipase-containing aqueous solution comprising lipase culture components and the like may be used. In the present invention, it is preferred to use lipase powders having a spherical shape and a water content of 10% by mass or less. Particularly, it is preferred that 90% by mass or more of the lipase powders have a particle size of 1 to 100 µm. Also, the lipase powders prepared by spray drying a lipase-containing aqueous solution adjusted to pH 6 to 7.5 are preferable.

In the present invention, it is preferred to use a granulated powder lipase which is granulated with a soybean powder and followed by powdering.

Here, as a soybean powder, it is preferred to use a soybean powder having a fat content of 5% by mass or more. In such soybean powder, the fat content is preferably 10% by mass or more and more preferably 15% by mass or more, while it is preferred that the fat content is 25% by mass or less. Specifically, the soybean powder having a fat content of 18 to 23 % by mass is preferred. In this connection examples of the fat contained in the soybean powder include fatty acid triglycerides and analogs thereof. The fat content in the soybean powder can be easily determined by means such as Soxhlet extraction method.

As such soybean powder, whole soybean flour can be used. Alternatively, as a raw material of the soybean powder, a soymilk can be used. The soybean powder can be prepared by grinding soybeans by conventional means. The soybean powders preferably have a particle size of approximately 0.1 to 600 µm. The particle size can be determined by means similar to that used in determining the particle size of the powdered lipase.

It is preferred that the powdered lipase has a water content of 10% by mass or less, and specifically a water content of 1 to 8 % by mass is preferred.

The particle size of the powdered lipases can be optional. However, 90% by mass or more of the powdered lipases preferably have a particle size of 1 to 100 µm. The powdered lipase preferably has an average particle size of 10 to 80 µm. The powdered lipase preferably has a spherical shape.

The powdered lipase used in the present invention can be prepared by drying an aqueous solution in which the lipase and the soybean powder are dissolved and dispersed by any of drying means selected from the groups consisting of spray drying, freeze drying and solvent precipitating followed by drying.

The aqueous solution in which the lipase and the soybean powder are dissolved and dispersed can be obtained by dissolving and dispersing the lipase powder and the soybean powder into a water, mixing the lipase powder with an aqueous solution in which the soybean powder is dissolved and dispersed, or mixing the soybean powder with a lipase-containing aqueous solution described below.

In the step of drying the aqueous solution in which the lipase and the soybean powder are dissolved and dispersed, particles of the lipase and/or the soybean powder agglutinate, and then a granulated substance comprising the lipase and the soybean powder is formed. The granulated substance may comprise lipase culture components.

The amount of water in the aqueous solution in which the lipase and the soybean powder are dissolved and dispersed is determined by adjusting the mass of the water to the total mass of the lipase and the soybean powder. Specifically, the mass of the water is preferably 0.5 to 1,000 times, more preferably 1.0 to 500 times and the most preferably 3.0 to 100 times of the total mass of the lipase and the soybean powder.

Particularly, when the powdered lipase is prepared by spray drying, the mass of water is preferably 2.0 to 1,000 times, more preferably 2.0 to 500 times and the most preferably 3.0 to 100 times of the total mass of lipase and soybean powder, because of character of devices for spray drying.

In the case of the lipase-containing aqueous solution is used as the raw material of the lipase, when the content of the lipase in the lipase-containing aqueous solution is unknown, the content of the lipase can be determined by freeze drying or other vacuum drying the lipase-containing aqueous solution to calculate a mass of the lipase.

Here, the aqueous solution containing the lipase includes a lipase culture solution from which cell bodies are removed, a purified culture solution thereof, a solution in which lipase obtained from these culture solutions is dissolved and dispersed again; a solution in which a commercially available powdery lipase is dissolved and dispersed again; and a commercially available liquid lipase. In order to enhance a lipase activity, it is more preferred that low-molecular-weight components such as salts are removed from the solution. In order to enhance the powder property, it is more preferred that low-molecular-weight components such as sugars are removed from the solution.

The granulated powdered lipase may contain the filter aid.

The powdered lipase described above can be used by itself for transesterification of the fat and oil, although the powdered lipase can be improved its lipase activity while purified by contacting with a long-chain fatty acid triglyceride(s) and a middle-chain fatty acid triglyceride(s) followed by collecting.

The long-chain fatty acid triglyceride used in doing so is preferred to be a triglyceride which its constituent fatty acids have 14 to 24 carbon atoms and particularly a vegetable oil selected from the group consisting of rapeseed oil, soybean oil, sunflower oil, safflower oil and corn oil.

The middle-chain fatty acid triglyceride is preferred to be a triglyceride which its constituent fatty acids have 6 to 12 carbon atoms. Such fatty acid triglycerides may be prepared by known method and be a commercially-available product. For example, the commercially-available product is provided from Nisshin OilliO Group, Ltd under the trade name of ODO.

It is preferred to use the long-chain fatty acid triglyceride and middle-chain fatty acid triglyceride in a mass ratio of 95:5 to 50:50, these triglycerides are preferred to be contacted with the lipase in the amount of 2 to 100 times of total mass of the lipase.

In the present invention, the above-described raw fat and oil is added the above-described lipase thereto and is conducted transesterification by conventional means. In this case, it is preferred that 0.01 to 10 parts by mass (preferably 0.01 to 2 parts by mass, and more preferably 0.05 to 1 parts by mass) of the powder lipase is added to the material based on 100 parts by mass thereof and transesterification is conducted at a temperature of 35 to 100 °C (preferably 40 to 80 °C, more preferably 50 to 80 °C and the most preferably 50 to 75 °C) for 0.1 to 50 hours (preferably 0.1 to 20 hours and more preferably 0.5 to 16 hours, or 0.1 to 3 hours are preferred, 0.5 to 2.5 hours are also preferred). The reaction is preferably conducted in batch reaction. The reaction temperature may be any temperature at which the reactive substrate fat and oil can melt and enzyme can be activated. The optimal reaction time varies depending on additive amount of oxygen, reaction temperature and the like.

After transesterification, a conventional fractionating step is conducted to remove high melting point triglycerides and low melting point triglycerides. The fractionating step may be conducted with or without solvent. There is explained the fractionation of the high melting point triglycerides. For the fractionation without solvent, it is preferred to cool to a temperature of 20 to 40 °C and remove the resultant solid content. Examples of the solvent used in solvent fractionation include acetone, hexane, ethanol, hydrous ethanol and the like, acetone and hexane are preferable and particularly acetone is preferred. Here, it is preferred that 50 to 1000 parts by mass of the solvent is added to 100 parts by mass of the transesterification product and it is cooled to a temperature of 10 to 40 °C to remove solid content (stearin part). Next, there is explained the removal of the low melting point triglycerides. The removal of the low melting point triglycerides may be conducted with or without solvent but preferably with the solvent. Examples of the solvent used in solvent fractionation include acetone, hexane, ethanol, hydrous ethanol and the like, acetone and hexane are preferable and particularly acetone is preferred. It is preferred that 50 to 1000 parts by mass of the solvent is added to 100 parts by mass of the transesterification product or liquid portion from which the high melting point fraction is removed (free from the solvent) and cooled to a temperature of -5 to 20 °C to remove the resultant solid content (stearin part). The steps of removing the high melting point fraction and removing the low melting point fraction, whichever may be conducted in first, although it is preferred that the step of removing the high melting point fraction is conducted in first. When removing the high melting point fraction is followed by removing the low melting point fraction, the liquid portion (olein part) obtained by the solvent fractionation may be subjected to distilling away the solvent therefrom and then added a solvent thereto again to fractionate, or the liquid portion obtained by the solvent fractionation may be continuously subjected to the fractionating step. However, it is preferred that the liquid portion obtained (olein part) is further cooled to a temperature of -5 to 20 °C without desolvation to obtain the solid content (stearin part).

When the raw material oil conducted transesterification is a fat and oil substantially free from the high melting point fraction (SSS) or the low melting point fraction (SU₂, S₂L), the fat and oil after the transesterification contains only a small amount of the high melting point fraction or the low melting point fraction and thus the fractionating step for the removal of the high melting point fraction and/or low melting point fraction can be omitted. The phrase "substantially free from" means not more than 1% by mass for the high melting point fraction and not more than 7% by mass for the low melting point fraction.

Thus, the present invention can provide a hard butter which contains 1,3-dipalmitoyl-2-oleoyl glycerin(POP) not less than 70% by mass, palmitoyl oleoyl stearoyl glycerin (POSt) not less than 5% by mass, tripalmitoyl glycerin (PPP) not more than 3% by mass, dipalmitoyl monolinoleyl glycerin (P₂L) not more than 5% by mass and monopalmitoyl dioleoyl glycerin (P₂O) not more than 3% by mass, and wherein a total content of POP and POSt is not less than 80% by mass and a mass ratio of POP/PPO is not less than 9/1.

In the present invention, furthermore, POP is preferred to be not less than 75% by mass. PPP is preferred to be not more than 2% by mass. P₂L is preferred to be not more than 3% by mass. P₂O is preferred to be not more than 2% by mass. Additionally, the total content of POP and POSt is preferred to be not less than 87% by mass, the mass ratio of POP/PPO is preferred to be 95/5 to 99.9/0.1. Particularly, it is preferred that the mass ratio of POP/PPO is 97/3 to 99.9/0.1 and more preferably 98/2 to 99.9/0.1.

In addition, in the present invention, it is preferred to substantially remove monopalmitoyl dioleoyl glycerin (PO₂) and/or tripalmitoyl glycerin (PPP) by fractionation after the transesterification described above. Here, in the present invention, the phrase "substantially remove" means that a substance to be removed is made not more than 5% by mass and preferably not more than 3% by mass. When the fat and oil substantially free from the high melting point fraction (SSS) and the low melting point fraction (SU₂, S₂L) is used as the raw material fat and oil, the fractionation after the transesterification can be also omitted. This phrase "substantially free from" means not more than 1% by mass for the high melting point fraction (SSS) and not more than 7% by mass, preferably not more than 5% by mass for the low melting point fraction (SU₂, S₂L).

Examples of the raw material fat and oil used to prepare the fat and oil having 9/1 or more of the SUS/SSU mass ratio in the present invention include fats and oils 1,3-disaturated long-chain fatty acid 2-monounsaturated long-chain fatty acid triglyceride (SUS) of 50% by mass. Among these, the fat and oil wherein the saturated long-chain fatty acids are palmitic acids or stearic acids is preferred, that wherein the saturated long-chain fatty acids are palmitic acids is more preferred, that wherein the monounsaturated fatty acids are oleic acids is preferred. Particularly, it is preferred that the fat and oil is derived from palm oil, more specifically palm midfraction is preferred. Alternatively, the fat and oil containing 50% by mass or more of dipalmitoyl monooleoyl glycerin is preferred. In addition, SSU includes 1-monounsaturated long-chain fatty acid-2,3-disaturated long-chain fatty acid triglyceride (USS) also. The fat and oil substantially free from the high melting point fraction (SSS) and the low melting point fraction (SU₂, S₂L) can omit the fractionation steps and is preferred.

Also, the lipases described above can be used to prepare the fat and oil having 9/1 or more of the SUS/SSU mass ratio of the present invention.

Chocolate products of the present invention comprise a fat and oil component and a sugar component, the fat and oil component being a mixture of the hard butter described above and cacao butter. It is preferable that the hard butter described above is incorporated into the fat and oil component at 10% by mass or more, preferably at 20% by mass or more, and the most preferably at 30% by mass or more. For the sugar component, any sugars usually used in chocolates can be used. For example, sucrose, fructose and mixture thereof are included. Sugar alcohols such as sorbitol can be used also. Furthermore, the chocolate products of the present invention can comprise other optional components comprised in conventional chocolate products. Examples of the optional components include emulsifying agent which is typically lecithin, flavoring, powdered skim milk, powdered whole milk and the like.

According to the present invention, a hard butter having improved POP/PPO mass ratio and excellent properties can be simply obtained for a short time by conducting transesterification of the fat and oil containing POP of 50% by mass or more and PPO which its raw material is palm oil stably supplied with an action of the specific 1,3-selective lipase. Thus the present invention has great merits from the viewpoint of industry.

Additionally, the hard butter obtained as described above can be used to produce various confectionaries, breads and the like. Particularly in combination with cacao butters and sweeteners, the hard butter can provide excellent chocolate products having an enhanced snap property and a good melting property in the mouth.

Next, the present invention will be explained in detail by the following

### Examples.

### Preparation example 1 [Preparing powdered lipase composition]

5 g of Lipozyme TL-IM (Novozymes A/S) was ground with L-type mycolloider (Tokushu kika kogyo Co., Ltd). The ground lipases were measured with Particle Size Distribution Analyzer LA-500 (HORIBA, Ltd), their average particle size was 66.4 µm. The powdered lipase was added 5 g of cellulose powders (NIPPON PAPER CHEMICALS Co., Ltd: average particle size 30 µm) thereto, and a powder composition was obtained. 5 g of the powder composition was added 90 g of rapeseed oil and 10 g of ODO (Nisshin OilliO Group, Ltd) thereto, the resultant was stirred at 25°C for 5 hours and then filtrated. As the result, a powdered lipase composition was obtained.

### Preparation example 2 [Preparing powdered lipase composition 2]

An aqueous solution of 10% deodorized whole fat soybean flour, which is sold under the trade name of "Alphaplus HS-600" manufactured by Nisshin Cosmo Foods Ltd. and has 23% fat content by mass, was previously autoclave sterilized for 15 minutes at 121°C and cooled to the order of room temperature. Triple amount of the cooled solution was added to an enzyme solution of Lipase DF "Amano" 15-K manufactured by Amano Enzyme Inc. and also referred to as Lipase D (150000 U/ml) with stirring. The resulting solution was adjusted to pH 7.8 with 0.5N NaOH solution and then spray dried with spray dryer (SD-1000: Tokyo Rikakikai Co., Ltd). As the result, a powdered lipase composition 2 was obtained.

### Comparative Example 1

To 10g of palm midfraction 1 (Composition: 62.3% by mass of POP, 11.9% by mass of PPO), there was added 5% by mass of Lipozyme RM-IM (immobilized lipase derived from *Mucor miehei*: Novozymes A/S) and then reacted with stirring for 4 hours at 70°C. The resulting solution was filtrated to remove the immobilized enzyme, and as the result, 9.5 g of reaction product 1 was obtained.

### Example 1

To 10 g of palm midfraction 1 (Composition: 62.3% by mass of POP, 11.9% by mass of PPO) which is same as used in Comparative Example 1, there was added 1.5% by mass of Lipozyme TL-IM (immobilized lipase derived from *Thermomyces* sp.: average particle size 800 µm) manufactured by Novozymes A/S and then reacted with stirring for 4 hours at 70°C. The resulting solution was filtrated to remove the immobilized enzyme, and as the result, 9.6 g of reaction product 2 was obtained.

### Example 2

To 10 g of palm midfraction 1 (Composition: 62.3% by mass of POP, 11.9% by mass of PPO) which is same as used in Comparative Example 1, there was added 0.2% by mass of the powdered lipase composition prepared in Preparation Example 1 and then reacted with stirring for 2 hours at 70°C. The resulting solution was filtrated to remove the powdered enzyme, and as the result, 9.5 g of reaction product 3 was obtained.

### Example 3

To 10 g of palm midfraction 1 (Composition: 62.3% by mass of POP, 11.9% by mass of PPO+OPP) which is same as used in Comparative Example 1, there was added 0.1% by mass of the powdered lipase composition 2 prepared in Preparation Example 2 and then reacted with stirring for 4 hours at 70°C. The resulting solution was filtrated to remove the powdered enzyme, and as the result, 9.6 g of reaction product 4 was obtained.

Table 1 shows compositions and decreased amounts of P₂O regarding the reaction products 1 to 4 obtained in Comparative Example 1 or Examples 1 to 3. Table 2 shows conditions for enzymatic reactions.

TAG compositions were analyzed with GLC (GC-2010: Shimadzu Corp.). Analysis method of TAG composition is explained in more detail. Conditions for GLC analysis are shown below.
Column: Rtx-65TG (Restek Corp.) 15 m×0.1 µm×0.25 mm
Detector: FID
Carrier Gas: He
Sprit Ratio: 60:1
Column Temperature (°C): 350°C (1 min) → (1°C /min) → 365°C (4 min)
Inlet Temperature: 365°C
Detector Temperature: 365°C

When these reaction progresses are compared in terms of a generated amount of P₃ and the decreased amount of P₂O, the reaction products 1 to 4 are thought that their reactions almost equivalently progress. When the reactions are compared in terms of the additive amount of enzyme and the enzymatic reaction time, the reaction time in Example 1 is same and the amount of enzyme in Example 1 is decreased to about 1/3 as compared to Comparative Example 1. The amount of enzyme in Example 2 is decreased to 1/25 and the reaction time in Example 2 is decreased to 1/2. The reaction time in Example 3 is same and the amount of enzyme in Example 3 is decreased to 1/50. From these findings, the reaction efficiencies of Examples 1 to 3 are extremely good as compared to that of Comparative Example 1.

**Table 1. TAG compositions**

| TAG composition(%) | Palm midfraction1 | Reaction product 1 (Comparative example 1) | Reaction product 2 (Example 1) | Reaction product 3 (Example 2) | Reaction product 4 (Example 3) |
|---|---|---|---|---|---|
| ppp | 2.3 | 9.8 | 10.4 | 10.0 | 7.0 |
| P₂St | 0.3 | 1.5 | 1.7 | 1.8 | 1.7 |
| P₂O | 74.2 | 58.3 | 59.2 | 60.1 | 59.4 |
| P₂L | 6.0 | 4.9 | 4.7 | 4.7 | 5.5 |
| PSt₂ | tr | 0.2 | 0.2 | 0.2 | 0.2 |
| Post | 10.9 | 9.2 | 9.1 | 9.2 | 10.1 |
| PO₂ | 1.3 | 9.1 | 8.9 | 8.3 | 8.1 |
| St₂O | 0.9 | 0.8 | 0.7 | 0.8 | 1.0 |
| StO₂ | tr | 0.6 | 0.4 | 0.4 | 0.6 |
| others | 4.1 | 5.6 | 4.7 | 4.5 | 6.4 |
| Reduced P₂O amount of P₂O | - | 15.9 | 15.0 | 14.1 | 14.8 |

| | | | | | |
|---|---|---|---|---|---|
| P: palmitic acid, St: stearic acid, O: oleic acid, L: linoleic acid, tr: trace | | | | | |

**Table 2. Comparison of reaction conditions**

| | Comparative example 1 | Example 1 | Example 2 | Example 3 |
|---|---|---|---|---|
| Enzyme concentration | 5.0% | 1.5% | 0.2% | 0.1% |
| Reaction time | 4 hours | 4 hours | 2 hours | 4 hours |
| Reaction temperature | 70 °C | 70 °C | 70 °C | 70 °C |

### Example 4

To 1200 g of palm midfraction 1 (Composition: 62.3% by mass of POP, 11.9% by mass of PPO) which is same as used in Comparative Example 1, there was added 0.2% by mass of the powdered lipase composition prepared in Preparation Example 1 and then reacted with stirring for 2 hours at 70°C. The resulting solution was filtrated to remove the powdered enzyme, and as the result, 1184 g of reaction product 5 was obtained. The obtained reaction product 5 (1132 g) was dissolved by adding 5660 g of acetone thereto. The resulting solution was cooled to 20°C, and then a generated solid phase was removed therefrom. The resulting liquid portion was cooled to 5°C, and then a generated solid phase was obtained by filtration therefrom. The resulting solid portion was subjected to removal of acetone and purification by conventional means, and as the result, 792 g of hard butter 1 (HB 1) was obtained.

TAG compositions of the raw material palm midfraction and the hard butter 1 are shown in Table 3, their POP/PPO ratios are shown in Table 4 (% described in Table represents % by mass). The TAG compositions were analyzed with GLC (GC-2010: Shimadzu Corp.), the POP/PPO ratios were analyzed with LC-MS/MS (Quattro micro: Nihon Waters K. K.).

The method of analyzing POP/(PPO+OPP) with LC-MS/MS will be explained in more detail. Analysis conditions for LC-MS/MS are listed below.
Column: conventional ODS columns (4.6 mm x 25 cm) dual linkage
Elution solvent: acetone/acetonitrile mixture
80/20 - 100/0 gradient mode
Flow Rate: 0.5 mL/min
Ion Source: APCI
Mass Analysis Part: MRM mode

The POP/(PPO+OPP) ratios were calculated by using a calibration curve drawn from reference materials of POP and PPO (Funakoshi Co., Ltd.). POP and PPO were mixed in various ratios, and the resultants were subjected to LC-MS/MS under the conditions described above. Ratios of Product ions M/Z=551 (PO) and M/Z=557 (PP) of M/Z=833 (P₂O), i.e., values of PO/PP were calculated, the calibration curve was obtained between the values and a value of a ratio of standard mixing of POP and PPO. Just like the reference materials, the samples were analyzed under the conditions described above to obtain PO/PP ratios, and their POP/(PPO+OPP) ratios were calculated from the calibration curve.

**Table 3. TAG compositions**

| TAG composition (%) | Palm midfraction 1 | Hard butter 1 |
|---|---|---|
| PPP | 2.3 | 1.8 |
| P₂St | 0.3 | 0.3 |
| P₂O | 74.2 | 79.7 |
| P₂L | 6.0 | 1.3 |
| PSt₂ | tr | tr |
| POSt | 10.9 | 12.6 |
| PO₂ | 1.3 | 0.2 |
| St₂O | 0.9 | 1.1 |
| StO₂ | tr | tr |
| Others | 4.1 | 3.0 |

**Table 4. POP/(PPO+OPP) ratios (mass ratio)**

| | Palm midfraction 1 | Hard butter 1 |
|---|---|---|
| POP/(PPO+OPP) | 84/16 | 97/3 |

### Example 5

To 700 g of palm midfraction 2 (Composition: 65.2% by mass of POP, 5.7% by mass of PPO+OPP), there was added 0.3% by mass of the powdered lipase composition prepared in Preparation Example 2 and then reacted with stirring for 8 hours at 50°C. The resulting solution was filtrated to remove the powdered enzyme, and 693 g of reaction product 6 was obtained. 650 g of the obtained reaction product 6 was measured out and dissolved by adding 3250 g of acetone thereto. The resulting solution was cooled to 20°C, and then a generated solid phase was removed therefrom. The resulting liquid portion was further cooled to 0°C, a generated solid phase was obtained by filtration therefrom, and then the solid portion was subjected to removal of acetone and purification by conventional means, and as the result, 435 g of hard butter 2 (HB2) was obtained.

TAG compositions and SOS/(SSO+OSS) ratios of the raw material palm midfraction 2 and the hard butter 2 are shown in Table 5 (% described in Table represents % by mass). The SOS/(SSO+OSS) ratios were analyzed by HPLC method with Chromspher Lipids column. Those were analyzed in isocratic mode with UV (205 nm) as the detector and 0.5% acetonitrile-hexane solution as the elution solvent.

There is explained the symbols used here. S means a saturated long-chain fatty acid and primarily palmitic acid or stearic acid. For example, SOS means a sum of POP, POSt, StOP and StOSt.

**Table 5. TAG compositions and SOS/(SSO+OSS) ratios**

| TAG composition(%) | Palm midfraction 2 | Hard butter 2 |
|---|---|---|
| PPP | 2.0 | 1.6 |
| P₂St | 0.3 | 0.2 |
| P₂O | 70.9 | 76.9 |
| P₂L | 6.0 | 2.9 |
| PSt₂ | tr | tr |
| POSt | 12.0 | 13.2 |
| PO₂ | 2.3 | 0.7 |
| St₂O | 1.2 | 1.3 |
| StO₂ | tr | tr |
| Others | 5.3 | 3.2 |
| SOS/SSO+OSS) | 92/8 | 98/2 |

| | | |
|---|---|---|
| S: saturated long-chain fatty acid (primarily, palmitic acid, stearic acid) | | |

### Example 6

To 350 g of palm midfraction 3 (Composition: 73.1% by mass of POP, 4.6% by mass of PPO+OPP), there was added 0.3% by mass of the powdered lipase composition prepared in Preparation Example 2 and then reacted with stirring for 2 hours at 50°C. The resulting solution was filtrated to remove the powdered enzyme, and 337 g of reaction product 7 was obtained. The obtained reaction product 7 was subjected to dry fractionation to remove the high melting point fraction rich in PPP, and the resultant low melting point fraction was purified. As the result, 435 g of hard butter 3 (HB3) was obtained.

TAG compositions and SOS/(SSO+OSS) ratios of the raw material palm midfraction 3 and the hard butter 3 are shown in Table 6 (% described in Table represents % by mass). The SOS/(SSO+OSS) ratios were analyzed by HPLC method with Chromspher Lipids column. Those were analyzed in isocratic mode with UV (205 nm) as the detector and 0.5% acetonitrile-hexane solution as the elution solvent.

**Table 6. TAG compositions and SOS/(SSO+OSS) ratios**

| TAG composition (%) | Palm midfraction 3 | Hard butter 3 |
|---|---|---|
| PPP | 2.8 | 1.2 |
| P₂St | 0.7 | 0.2 |
| P₂O | 77.7 | 75.8 |
| P₂L | 3.0 | 3.0 |
| PSt₂ | tr | tr |
| POSt | 12.5 | 12.9 |
| PO₂ | 0.4 | 2.6 |
| St₂O | 1.1 | 1.3 |
| StO₂ | tr | 0.1 |
| Others | 1.8 | 2.9 |
| SOS/(SSO+OSS) | 94/6 | 99/1 |

Figure 1 and Table 8 show results of analyzing solid fat contents in formulations of cacao butter with the palm midfraction 1, the palm midfraction 3 or the hard butter 1. These formulations shown in Table 7 were analyzed.

**Table 7. Formulas of fats and oils for analyzing solid fat contents**

| | Formulation 1 | Formulation 2 | Formulation 3 |
|---|---|---|---|
| Palm midfraction 1 | | | 15 |
| Palm midfraction 3 | | 15 | |
| Hard butter 1 | 15 | | |
| Cacao butter 1 | 85 | 85 | 85 |

**Table 8. Solid fat contents (SFC)**

| | Solid fat content (%) | | | | |
|---|---|---|---|---|---|
| | 25.0 °C | 27.5 °C | 30.0 °C | 32.5°C | SFC difference |
| Formulation 1 | 51.3 | 37.9 | 5.8 | 0 | 45.5 |
| Formulation 2 | 49.8 | 36.1 | 6.2 | 0 | 43.6 |
| Formulation 3 | 47.9 | 35.6 | 6.5 | 0 | 41.4 |

### SFC difference: difference between SFC at 27.5°C and SFC at 32.5 °C

The formulation 1 comprising the hard butter 1 had the highest solid fat content at 27.5°C. However, the formulation 1 had the lowest fat content at 32.5°C and the biggest SFC difference between SFC at 27.5°C and SFC at 32.5 °C. From these findings, it is thought that the formulation 1 is a fat and oil which is best in snap property and melting property in the mouth.

### Example 7

A chocolate was prepared experimentally with the above-described hard butter (the hard butter 1, the palm midfraction 1) in the formula shown in Table 9 and evaluated. There was no special trouble on its viscosity and demolding property at the time of preparing the chocolate. The obtained chocolate was evaluated regarding snap property, luster and melting property in the mouth after storage for a week at 20°C. As the result, chocolate 1 using the hard butter 1 had a good melting property in the mouth and excellent snap property.

**Table 9. Formula of chocolate (% by mass)**

| | Comparative chocolate 1 | Control chocolate 2 | Chocolate 1 |
|---|---|---|---|
| Sugar | 43.45 | 43.45 | 43.45 |
| Cacao mass | 40.0 | 40.0 | 40.0 |
| *( Cacao butter content) | (22.0) | (22.0) | (22.0) |
| Cacao butter | 16.0 | 1.0 | 1.0 |
| Hard butter 1 | | | 15.0 |
| Palm midfraction 1 Lecithin | | 15.0 | |
| Flavoring | 0.5 | 0.5 | 0.5 |
| | 0.05 | 0.05 | 0.05 |

### [Evaluation result of chocolate]

Chocolates prepared by the process described above were evaluated regarding demolding property, snap property, luster, and melting property in the mouth. The evaluation result is shown in Table 10.

**Table 10. Evaluation results of bars of chocolates**

| | Comparative chocolate 1 | Comparative chocolate 2 | Chocolate 1 |
|---|---|---|---|
| Snap property | ○ | △ | ⊚ |
| Melting property in the mouth | ○ | ⊚ | ⊚ |
| Demolding property | ⊚ | ○ | ⊚ |
| Luster | ⊚ | ○ | ⊚ |

These chocolates were evaluated with sensory test by ten panelists.

### Evaluation criteria are described below.

### [Evaluation criteria]

- Snap property: ⊚: very good snap property
○: good snap property
Δ: inferior snap property

- Melting property in the mouth: ⊚: very good melting property in the mouth
○: good melting property in the mouth
Δ: but melting property in the mouth

- Luster: ⊚: very good
○: good, but dulling is partly shown.
Δ: dulling

- Demolding property: ⊚: It can be remolded after refrigeration for15 minutes.
○: It can be remolded after refrigeration for 20 minutes.
Δ: It can not be remolded.

## Claims

1. A process for preparing a fat and oil having a 1,3-disaturated long-chain fatty acid 2-monounsaturated long-chain fatty acid triglyceride (SUS)/1,2-disaturated long-chain fatty acid 3-monounsaturated long-chain fatty acid triglyceride (SSU) mass ratio of 9/1 or more, which comprises conducting transesterification of a fat and oil containing SUS of 50% by mass or more and SSU with an action of a 1,3-selective lipase selected from the group consisting of lipases derived from *Thermomyces* sp., *Rhizopus oryzae* and *Rhizopus delemar*.

2. The process according to claim 1, wherein the lipase is derived from *Thermomyces* sp.

3. The process according to claim 1 or 2, which comprises removing the resultant diunsaturated long-chain fatty acid monosaturated fatty acid triglyceride (SU₂) and/or trisaturated fatty acid triglyceride (SSS) by fractionation after the transesterification.

4. The process according to any of claims 1 to 3, wherein the saturated long-chain fatty acid is palmitic acid.

5. The process according to any of claims 1 to 4, wherein the unsaturated long-chain fatty acid is oleic acid.

6. The process according to any of claims 1 to 5, wherein the fat and oil containing 1,3-disaturated long-chain fatty acid 2-monounsaturated long-chain fatty acid triglyceride (SUS) and 1,2-disaturated long-chain fatty acid 3-monounsaturated long-chain fatty acid triglyceride (SSU) is derived from palm oil.

7. The process according to claim 6, wherein the fat and oil containing 1,3-disaturated long-chain fatty acid 2-monounsaturated long-chain fatty acid triglyceride (SUS) and 1,2-disaturated long-chain fatty acid 3-monounsaturated long-chain fatty acid triglyceride (SSU) is palm midfraction.

8. The process according to any of claims 1 to 7, wherein the fat and oil containing 1,3-disaturated long-chain fatty acid 2-monounsaturated long-chain fatty acid triglyceride (SUS) and 1,2-disaturated long-chain fatty acid 3-monounsaturated long-chain fatty acid triglyceride (SSU) contains 50% by mass or more of dipalmitoyl monooleoyl glycerin.

9. A process for preparing a hard butter, which comprises conducting transesterification of a fat and oil containing 1,3-dipalmitoyl-2-oleoyl glycerin (POP) of 50% by mass or more and 1,2-dipalmitoyl-3-oleoyl glycerin (PPO) with an action of a 1,3-selective lipase selected from the group consisting of lipases derived from *Thermomyces* sp., *Rhizopus oryzae* and *Rhizopus delemar*.

10. The process according to claim 9, which comprises substantially removing monopalmitoyl dioleoyl glycerin (PO₂) and/or tripalmitoyl glycerin (PPP) by fractionation after the transesterification.

11. The process according to any of claims 1 to 10, wherein the lipase derived from *Thermomyces* sp. is immobilized on a carrier.

12. The process according to any of claims 1 to 11, wherein the lipase derived from *Thermomyces* sp. is a powdered lipase.

13. The process according to claim 12, wherein the powdered lipase is obtained by grinding a lipase immobilized on a carrier.

14. The process according to claim 11 or 13, wherein the carrier is silica.

15. The process according to claim 1, the lipase derived from *Rhizopus ozyzae* or *Rhizopus delemar* is formed into a granulated powdered lipase containing the said lipase and a soybean powder.

16. The process according to any of claims 1 to 15, wherein the lipase is a lipase composition containing the powdered lipase and a filter aid.

17. The process according to any of claims 12 to 16, wherein the powdered lipase has an average particle size of 1 to 200 µm.

18. The process according to any of claims 1 to 17, wherein the mass ratio of SUS/SSU is not less than 95/5.
